Europäisches Patentamt

European Patent Office

Office européen des brevets

(19)

(11) Veröffentlichungsnummer: **0 175 352 B1**

## (12) EUROPÄISCHE PATENTSCHRIFT

(45) Veröffentlichungstag der Patentschrift: **12.06.91**

(21) Anmeldenummer: **85111761.4**

(22) Anmeldetag: **17.09.85**

(51) Int. Cl.⁵: **G01N 21/64**, A61B 5/00, G01K 11/20

(54) **Verfahren und Anordnung zur schnellen Bestimmung der Parameter eines Probenmediums.**

(30) Priorität: **19.09.84 DE 3434423**

(43) Veröffentlichungstag der Anmeldung:
**26.03.86 Patentblatt 86/13**

(45) Bekanntmachung des Hinweises auf die
Patenterteilung:
**12.06.91 Patentblatt 91/24**

(84) Benannte Vertragsstaaten:
**CH DE FR GB IT LI NL SE**

(56) Entgegenhaltungen:
**EP-A- 0 091 390**
**US-A- 3 910 701**
**US-A- 4 003 707**
**US-A- 4 167 331**

**ADVANCES IN INSTRUMENTATION, Band 38,
Oktober 1983, Seiten 925-932, ISA, Chicago,
US; R.V. ALVES et al.: "Temperature sensing
using optical fibers"**

(73) Patentinhaber: **Siemens Elema AB**
**Röntgenvägen 2**
**S-171 95 Solna 1(SE)SE**

Patentinhaber: **Siemens Aktiengesellschaft**
**Wittelsbacherplatz 2**
**W-8000 München 2(DE)CH DE FR GB IT LI NL**

(72) Erfinder: **Brauer, Stefan**
**Bjoerkstigen 14**
**S-240 17 Soedra Sandby(SE)**
Erfinder: **Johansson, Jan**
**Gustav Snickars Vaeg**
**S-240 36 Stehag(SE)**
Erfinder: **Nilsson, Jan-Ove, Dr.**
**Trastvaegen 13b**
**S-222 31 Lund(SE)**
Erfinder: **Olsson, Sven-Gunnar**
**PL 652**
**S-240 17 Soedra Sandby(SE)**

## Beschreibung

Die Erfindung betrifft ein Verfahren zur schnellen Bestimmung der Parameter eines Probenmediums, insbesondere eines fliessenden Probenmediums, gemäss dem Oberbegriff des Anspruches 1 so- wie eine Anordnung zur Durchführung des Verfahrens. Ein derartiges Verfahren ist z.B. aus der DE-OS 3 148 830 bekannt.

Für zahlreiche Anwendungsgebiete ist es erforderlich, dass die Bestimmung beispielsweise der Sauerstoffkonzentration in Gasen, Flüssigkeiten und dergleichen, mit extrem kurzer Einstellzeit von wenigen ms und ohne Totzeit erfolgt und dass bei der Messung kein Sauerstoff verbraucht wird. Beispielsweise ist es bei der Respiratorbehandlung von Patienten wünschenswert, die Sauerstoffkonzentration in der Ausatmungsluft zu überwachen.

Für die Messung mit kurzen Einstellzeiten hat sich das Prinzip der Lumineszenzlöschung von mit Licht bestimmter Wellenlänge zur Lumineszenz angeregten Schichten als erfolgversprechend erwiesen. Untersuchungen haben nun gezeigt, dass die Lumineszenzeigenschaften der Schichten nicht nur durch Sauerstoff, sondern auch noch durch weitere Parameter beeinflusst werden. So ändert z.B. die Temperatur die Intensität des Lumineszenzlichtes in gleicher Weise wie Sauerstoff, d.h. mit steigender Temperatur nimmt die Amplitude des emittierten Lichtes ab. Darüberhinaus verursacht steigende Temperatur aber noch eine Wellenlängenverschiebung zu grösseren Wellenlängen hin. Weiterhin haben Feuchtigkeit oder Wasser einen Einfluss auf die Lumineszenzeigenschaften.

Im speziellen Fall der Respiratorbehandlung sind auch der Einfluss von Anästhesiegasen wie Lachgas oder Halothan von Interesse.

Ganz allgemein gibt es eine Vielzahl von Stoffen, die bestimmte Lumineszenzschichten beeinflussen. Oft wird es von der gewählten Kombination von Trägermaterial und Lumineszenzfarbstoff abhängen. Als Probenmedien kommen im wesentlichen fliessende Probenmedien wie Gase oder Flüssigkeiten in Betracht. Das schliesst jedoch andere Probenmedien nicht aus. Wesentlich für die Messung eines Parmeters mit Hilfe der Lumineszenzlöschung ist allein, dass die Lumineszenzschicht durch den zu bestimmenden Parameter des Probenmediums beeinflusst wird.

Die zusätzlichen Veränderungen des Lumineszenzlichtes durch andere Parameter wurden bisher als unüberwindbare Schwierigkeiten für die rasche Sauerstoffkonzentrationsmessung angesehen. Um den Einfluss von Feuchtigkeit zu beseitigen, wurden bisher die Schichten mit einer Membran gegen das Probenmedium abgeschirmt (US-PS 4 003 707). Dadurch wurde aber bei den verwendeten Schichten die Einstellzeit stark verlängert, so dass sich diese bekannte Messanordnung nicht für die rasche Bestimmung der Sauerstoffkonzentration z.B. in der Patientenüberwachung eignet.

Der Temperatureinfluss auf die Lumineszenzschichten wurde bisher praktisch nur für reine Temperaturfühler untersucht. Bei der Sauerstoffbestimmung in Atemgasen wurde der Temperatureffekt als störend angesehen und beispielsweise versucht zu beseitigen, indem das Atemgas vor der Analyse auf eine vorbestimmte Temperatur gebracht wurde. Unter Umständen wurde gleichzeitig auch eine bestimmte Feuchtigkeit eingestellt. Auch diese Massnahmen führen im Ergebnis wieder zu einer uperwünscht hohen Einstellzeit.

Aus der eingangs genannten DE-OS 3 148 830 sind zwar bereits Lumineszenzschichten mit wasserabstossendem Trägermaterial bekannt, deren Lumineszenzeigenschaften durch Feuchtigkeit nicht mehr beeinflusst werden, die Temperatureffekte bleiben jedoch bestehen.

Der vorliegenden Erfindung liegt die Aufgabe zugrunde, ein Verfahren der eingangs genannten Art anzugeben, dass trotz der angeführten Schwierigkeiten auch beim Vorliegen mehrerer die Lumineszenzeigenschaften beeinflussenden Parameter eine zuverlässige Bestimmung eines Parameters des Probenmediums mit extrem kurzer Einstellzeit ermöglicht.

Eine weitere Aufgabe der Erfindung besteht darin, neben diesem ersten Parameter gleichzeitig mindestens einen weiteren Parameter bestimmen zu können.

Diese Aufgabe wird erfindungsgemäss durch die im kennzeichnenden Teil des Anspruchs 1 angegebenen Merkmale gelöst.

Die Erfindung nutzt dabei gerade die in Fachkreisen als störend angesehenen Beeinflussungen der Lumineszenzeigenschaften durch unterschiedliche Parameter vorteilhaft aus, um die hinreichend schnelle Messung eines Parameters, z.B. der Sauerstoffkonzentration, überhaupt erst zu ermöglichen und um darüberhinaus sogar noch die Möglichkeit zu schaffen, einen oder mehrere weitere Parameter zu bestimmen. Im Gegensatz zum Stand der Technik wird hier also nicht mehr versucht, den Einfluss der weiteren Parameter zu unterdrücken. Im Gegensatz dazu wird hier die zusätzliche Lumineszenzbeeinflussung durch die weiteren Parameter ebenfalls bestimmt und zur Ermittlung des gesuchten Parameters herengezogen.

Zur Durchführung des Verfahrens muss die Intensität des Lumineszenzlichtes für mindestens soviele verschiedene Wellenlängenbereiche bestimmt werden, wie Parameter vorhanden sind, die die Lumineszenzeigenschaften beeinflussen. Gleichzeitig müssen die Wellenlängenbereiche so gewählt sein, dass wenigstens ein Parameter die Schichteigenschaften bei den verschiedenen Wellenlängenbereichen unterschiedlich beeinflusst. Um den Messaufwand und auch den Aufwand für die Bestimmung des Parameters aus den Messergebnissen in vertretbaren Grenzen zu halten, ist es dabei möglich, geschickt verschiedene Lumineszenzschichten auszuwählen, die auf unterschiedliche Parameterkombinationen reagieren und deren Eigenschaften unabhängig von einem Teil der Parameter sind. So ist es beispielsweise möglich, Lumineszenzschichten auszuwählen, deren Lumineszenzeigenschaften nur von zwei Parametern, beispielsweise von dem Sauerstoffgehalt und der Temperatur des Probenmediums abhängen. Kombiniert man eine derartige Schicht mit einer anderen Schicht, die zusätzlich noch durch Feuchtigkeit beeinflussbar ist, so lassen sich über die erste Schicht bereits zwei Parameter bestimmen, die dann für die zweite als bekannt vorausgesetzt werden können. Dadurch verringert sich der Aufwand, um auch den dritten Parameter zu ermitteln.

Aus der US-PS 4 003 707 ist bekannt, die Messung der Lumineszenzlöschung mehrfach bei verschiedenen Wellenlängen vorzunehmen. Dabei wird gleichzeitig die Wellenlänge des Anregungslichtes und die des detektierten Lumineszenzlichtes verändert. Die verschiedenen Messignale werden einer Signalbearbeitungseinrichtung zugeführt, die daraus jedoch wieder nur einen Parameter bestimmt und Störungen durch andere Parameter nicht berücksichtigt. Durch die verschiedenen Messungen werden lediglich optische Fehler des Systems, Streulichteffekte und Einzelmessungsfehler beseitigt.

Zur Erhöhung der Messgenauigkeit ist in Weiterbildung der Erfindung vorgesehen, die spektrale Verteilung und/oder die Intensität des auf die lumineszierende Schicht gelenkten Lichtes über einen Regelkreis konstant zu halten. Das ermöglicht die Verwendung einfacherer und damit insbesondere für eine Serienproduktion vorteilhafterer Lichtquellen wie z.B. Leuchtdioden (LED).

Zusätzlich oder alternativ ist vorgesehen, die Intensität des von der lumineszierenden Schicht kommenden Lichtes im Wellenlängenbereich des Anregungslichtes zu bestimmen und als Referenzsignal zur Korrektur der Lumineszenzintensitäten bei den ausgewählten Wellenlängenbereichen zu verwenden. Selbst wenn die Intensität der Lichtquelle konstant ist, so ist damit noch nicht sichergestellt, dass stets die gleiche Lichtmenge auf die Schicht fällt. Gerade bei den relativ kleinen Intensitätsunterschieden, die durch die zu bestimmenden Parameter hervorgerufen werden, ist es daher wichtig, alle Störeffekte optimal auszuschalten.

Es ist für das Verfahren vorteilhaft, wenn das auf die lumineszierende Schicht gelenkte Licht gepulst ist. Dadurch können u.a. Driften in den Detektoren und/oder der Signalbearbeitungseinrichtung vermieden werden, indem z.B. in den Pausen die entsprechenden Komponenten auf Null zurückgesetzt werden. Weiterhin wird dadurch insbesondere bei der Verwendung von LED's vermieden, dass diese sich zu sehr erwärmen und damit ein anderes Emissionsspektrum erhalten. Die Lichtimpulse können auch mittels einer konstant betriebenen Lichtquelle und eines optischen oder mechanischen Zerhackers erzeugt werden.

Für die Ermittlung zweier Parameter mit Hilfe der Signalbearbeitungseinrichtung wird vorteilhaft der Ansatz gemacht, dass die Detektorsignale $S_i$ sich aus einem Untergrundanteil, einem linear von einem der Parameter und einem reziprok vom anderen Parameter abhängenden Anteil zusammensetzen nach der Formel:

$$S_i = \frac{i_i}{1 + k_i X_1} + b_i X_2 + a_i$$

wobei $i_i$ die Lumineszenzintensität bei der Wellenlänge $\lambda_i$ und bei einem bestimmen Wert der Parameters $X_1$ ( insbesondere bei $X_1 = 0$), $a_i$ die Untergrundintensität und $k_i, b_i$ Näherungskoeffizienten sind. Der Koeffizient $k_i$ ist dabei ein Mass für die Lumineszenzlöschung durch den Parameter $X_1$, wenn keine anderen Parameter die Lumineszenz beeinflussen. Der Koeffizient $b_i$ gibt an, wie stark der Parameter $X_2$ das Signal $S_i$ beeinflusst. Der Index i läuft hierbei von 1-2, wenn mit zwei Lumineszenzwellenlängen gemessen wird.

Es ist zur Verbesserung der Messgenauigkeit jedoch möglich, mehr als zwei Wellenlängen zu verwenden. Lediglich der Aufwand für die Signalauswertung steigt damit an.

In Versuchen hat sich gezeigt, dass sich zwei Parameter mit Hilfe dieser Formel sehr genau bestimmen lassen. Bei mehr als zwei Parametern muss eine andere Formel aufgestellt werden.

Neben dieser Berechnungsmethode zweier Parameter aus den zwei Detektorsignalen ist es auch noch möglich, die Parameter iterativ aus den Detektorsignalen zu ermitteln, wie an Hand eines Beispieles später noch naher gezeigt werden wird.

Um eine den hohen Anforderungen an die Zuverlässigkeit insbesondere im Bereich der Patientenbeatmung gerecht werdende Anordnung zur Durchführung des erfindungsgemässen Verfahrens zu erhalten, ist vorgesehen, dass als Lichtquelle mindestens eine Leuchtdiode (LED) vorgesehen ist. Ein Parameter der LED wird bestimmt und derart zur Ansteuerung der Diode(n) herangezogen, dass die Temperatur des aktiven Diodenbereiches einen definierten Wert bzw. Verlauf erhält.Dadurch soll erreicht werden, dass die Intensität und die spektrale Verteilung des emittierten Lichtes sich möglichst nicht verändern. Um aus dem Emissionsspektrum der LED die richtige Anregungswellenlänge auszuwählen, ist zwischen der LED und der lumineszierenden Schicht mindestens ein Filter angeordnet.

Es sei an dieser Stelle angemerkt, dass, wenn es die Verhältnisse zulassen, gleichwertig auch andere Lichtquellen eingesetzt werden können. Wegen der hohen Intensität und der Monochromasie ist ein Laser immer eine ausgezeichnete Lichtquelle.Auf eingangsseitige Filter kann dann verzichtet werden. Ebenso sind Laser-dioden denkbar, wenn diese für die erforderlichen Anregungswellenlängen und mit genügender Stabilität und Lebensdauer zur Verfügung stehen.

Wenn die Intensität und gegebenenfalls auch die spektrale Verteilung des emittierten Lichtes schwanken, müssen u.U. diese Variationen bestimmt und zur Korrektur der Detektorsignale herangezogen werden.

Zwischen der Schicht und den Detektoren sind weitere Filter angeordnet. Wesentlich für die Anordnung ist auch, dass eine Vorrichtung vorgesehen ist , über die das Probenmedium direkt oder indirekt an die Schicht herangeführt wird.

Um bei der Verwendung von LED's die Intensität des Anregungslichtes zu erhöhen und u.U. Exemplarschwankungen einzelner LED's auszugleichen, ist es vorteilhaft, eine Anzahl in Reihe oder parallel geschalteter LED's vorzusehen. Gleichzeitig wird dadurch das Signal/Rausch-Verhältnis verbessert.

Die Leuchtdioden können dabei kontinuierlich betrieben werden, wobei zur Regelung beispielsweise der Spannungsabfall über den Leuchtdioden herangezogen wird und über diesen Wert der durch die Leuchtdioden fliessende Strom eingestellt wird. Um Driften in den verwendeten Bauelementen zu vermeiden, ist es dabei möglich, in willkürlich vorgebbaren Zeitabständen die Leuchtdioden kurzzeitig abzuschalten und in diesen Pausen sämtliche Komponenten abzugleichen.

Um insbesondere eine zu starke Erwärmung der Leuchtdioden zu vermeiden, ist in Weiterbildung der Erfindung vorgeshen, dass diese gepulst betrieben werden. Dabei kann es sich um Konstantstrom-Impulse handeln. In diesem Fall kann eine Regelung der Leuchtdioden über die Impulsdauer vorgenommen werden. Für diesen Fall ist vorgesehen, dass die Detektoren die Intensität des Lumineszenzlichtes nur während eines Teils dieser Impulsdauer registrieren. Die Länge des Anregungsimpulses bleibt dadurch ohne Einfluss auf die Messignale. Eine andere Möglichkeit der Regelung besteht darin, den Stromverlauf während der Impulse zu variieren.

In einer vorteilhaften Weiterbildung ist vorgesehen, dass die Intensität zweier Wellenlängenbereiche der Leuchtdioden gemessen und deren Quotient als Regelparameter verwendet wird.

Um auch noch andere mögliche Störeinflüsse wie verschmutzte Filter oder dergleichen zu eliminieren, ist vorgesehen, dass die Registrierzeit in Abhängigkeit von einem Referenzsignal geregelt wird. Als Referenzsignal kann dazu das von der Lumineszenzschicht kommende Licht im Wellehlängenbereich des Anregungslichtes dienen. Dazu ist es vorteilhaft, einen weiteren Detektor sowie ein Filter zwischen diesem und der Schicht vorzusehen. Das Messignal dieses Detektors kann direkt zur Regelung der Registrierzeit herangezogen werden. Dazu ist es möglich, das Signal des Detektors zu integrieren und die Registrierung beim Erreichen eines vorgegebenen Integrationswertes zu beenden. Das bedeutet praktisch eine automatische Normierung.

Eine andere Möglichkeit besteht darin, dass die Messignale der das Lumineszenzlicht verschiedener Wellenlänge registrierenden Detektoren durch das Signal des weiteren Detektors relativiert werden, d.h., dass jedes Messignal durch das Signal des weiteren Detektors dividiert wird, bevor es der Signalbearbeitungseinrichtung zugeleitet wird.

Eine vorteilhafte Weiterbildung des erfindungsgemässen Verfahrens mit gepulst betriebenen Leuchtdioden ergibt sich daraus, dass ein Parameter der Leuchtdioden während des Impulses und insbesondere zum Beginn des Impulses bestimmt und zur Formung desselben herangezogen wird und dass darüberhinaus in Abhängigkeit vom Wert dieses Parameters die zeitliche Lage der Messdauer, in der die Detektoren die Lumineszenzintensität messen, festgelegt wird. Misst man als Parameter wieder den Spannungsabfall über den Dioden zu Beginn des Impulses, so gibt das eine Aussage über die Temperatur der Leuchtdioden. In Abhängigkeit von dieser Temperatur stellt sich eine bestimmte spektrale Verteilung ein. Liegt diese Temperatur zu niedrig, so kann man den Impuls verlängern oder die Stromstärke während des Impulses erhöhen. Beides führt zu einer Erhöhung der Diodentemperatur. Liegt die Temperatur zu Anfang zu hoch,

EP 0 175 352 B1

so geht man den umgekehrten Weg. In beiden Fällen wird sich zu einem bestimmten Zeitpunkt innerhalb des Impulses eine Diodentemperatur einstellen, die der gewünschten spektralen Verteilung entspricht. Legt man in Abhängigkeit von dem am Anfang des Impulses gemessenen Parameter die Zeitspanne für die Lumineszenzmessung in den Bereiche, in dem die Leuchtdioden die gewünschte Temperatur und damit spektrale Verteilung aufweisen, so ergeben sich zumindest gemittelt stets gleiche Verhältnisse.

Um den Aufwand für die Vermeidung von Störeinflüssen herabzusetzen, ist in einer konstruktiven Ausgestaltung der Erfindung vorgesehen, dass die LED's, die Filter , die lumineszierende Schicht und die Detektoren in einem lichtdichten Gehäuse angeordnet sind.

Anhand von 5 Figuren wird im folgenden ein Ausführungsbeispiel der Erfindung näher beschrieben und erläutert. Dabei zeigt bzw. zeigen

Fig. 1     schematisch den prinzipiellen Aufbau einer Anordnung zur Bestimmung zweier Parameter,

Fig. 2     den zeitlichen Verlauf der Einschaltzeiten der LED's und der Detektoren gemäss Fig.1,

Fig.3     ein Emissionsspektrum für eine lumineszierende Schicht und

Fig. 4,5     schematische Detektorsignalverläufe für zwei Wellenlängen in Abhängigkeit vom Sauerstoffpartialdruck und von der Temperatur.

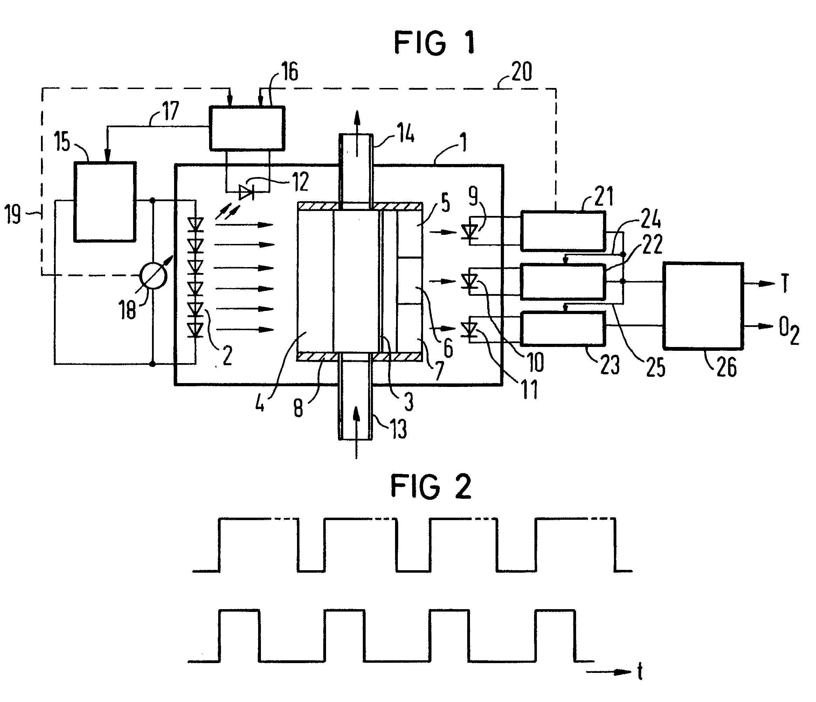

Fig. 1 zeigt schematisch und teilweise im Schnitt eine Anordnung zur gleichzeitigen Bestimmung der Sauerstoffkonzentration und der Temperatur eines Gases. In einem lichtdichten Gehäuse 1 befinden sich eine Anzahl in Reihe geschalteter LED's 2, eine transparente lumineszierende Schicht 3 und Filter 4-7, die zusammen mit der Schicht in einem Halter 8 angeordnet sind, sowie vier Fotodioden 9-12. Das Gehäuse 1 und der Halter 8 sind mit einem Gaseinlass 13 und einem Gasauslass 14 versehen. Die Richtung des Gasstromes ist durch Pfeile angedeutet. Sie ist ohne Bedeutung für das Funktionieren der Anordnung. Das Gas wird auf diese Weise direkt an der Lumineszenzschicht vorbeigeleitet.In Fig. 1 ist das nur prinzipiell und nicht massstäblich angedeutet. Damit der Parameter, der gemessen werden soll, schnell an der Schicht 3 variiert werden kann, sollten Gasein- bzw. Gasauslass gross und das Volumen zwischen Filter 4 und Schicht 3 dagegen klein sein.

Die LED's werden über eine Stromversorgung 15 gepulst betrieben. Im oberen Teil der Fig.2 ist der zeitliche Stromverlauf dargestellt, wenn mit konstantem Strom gepulst wird. Gestrichelt ist dabei der Teil der Impulse dargestellt,der variiert werden kann. Es ist im Bedarfsfall, wie bereits eingangs angegeben, auch möglich, andere Impulsformen zu wählen. Die Regelung erfolgt über die Stromversorgung 15 in Abhängigkeit von einem Diodenparameter. In der Fig 1 sind alternativ verschiedene Möglichkeiten angedeutet. So kann über den Detektor 12 die Intensität des ausgestrahlten Lichtes bestimmt und einer Servovorrichtung 16 zugeführt werden, die wiederum , wie durch den Pfeil 17 angedeutet, die Stromversorgung 15 ansteuert.

Eine weitere Möglichkeit besteht darin, den Spannungsabfall über den LED's mit einem Messinstrument 18 zu bestimmen und dieses Signal der Servovorrichtung zur Steuerung zuzuführen, wie gestrichelt durch die Leitung 19 angedeutet ist.

Eine dritte Möglichkeit besteht darin, die Intensität des durch die Lumineszenzschicht und ein an den Wellenlängenbereich des Anregungslichtes angepasstes Filter hindurchgehendes Licht mittels des Detektors 9 zu messen und dieses Signal, wie durch die gestrichelte Leitung 20 angedeutet, wiederum der Servovorrichtung zuzuführen.

Unabhängig von der Art der Regelung kann damit erreicht werden, dass die spektrale Verteilung und die Intensität des emittierten Lichtes konstant bleiben oder zumindest einen definierten Verlauf haben.

6 und 7 sind zwei Filter für unterschiedliche Wellenlängenbereche des Lumineszenzspektrums. Das durch diese Filter hindurchgehende Licht wird durch die Detektoren 10 bzw. 11 registriert.

An die Detektoren 9-11 sind unmittelbar Verstärker 21-23 angeschlossen,die zusätzlich möglicherweise Integrations-, Differentier-und andere Glieder enthalten können. Sie bestimmen gleichzeitig die Impulsdauer für die Registrierung der von den Detektoren kommenden Signale, wie sie im unteren Teil der Fig 2 dargestellt ist.

Wird mit dem Verstärker 21 das Signal des Detektors 9 bestimmt, so kann dieses über Leitungen 24,25 auf die Verstärker 22 bzw. 23 gegeben werden, in denen dann der auf die Intensität des Anregungslichtes normierte Signalwert der Detektoren 10 bzw 11 gebildet wird.

Wie im unteren Teil der Fig.2 dargestellt, ist die Impulsbreite für die Registrierung des Lumineszenzlichtes kleiner als die kleinste Impulsbreite des Anregungslichtes.

Die Ausgangssignale der beiden Verstärker 22 und 23 sind auf eine Signalbearbeitungseinrichtung 26 gegeben, die daraus die Werte für die Sauerstoffkonzentration und die Temperatur ermittelt.

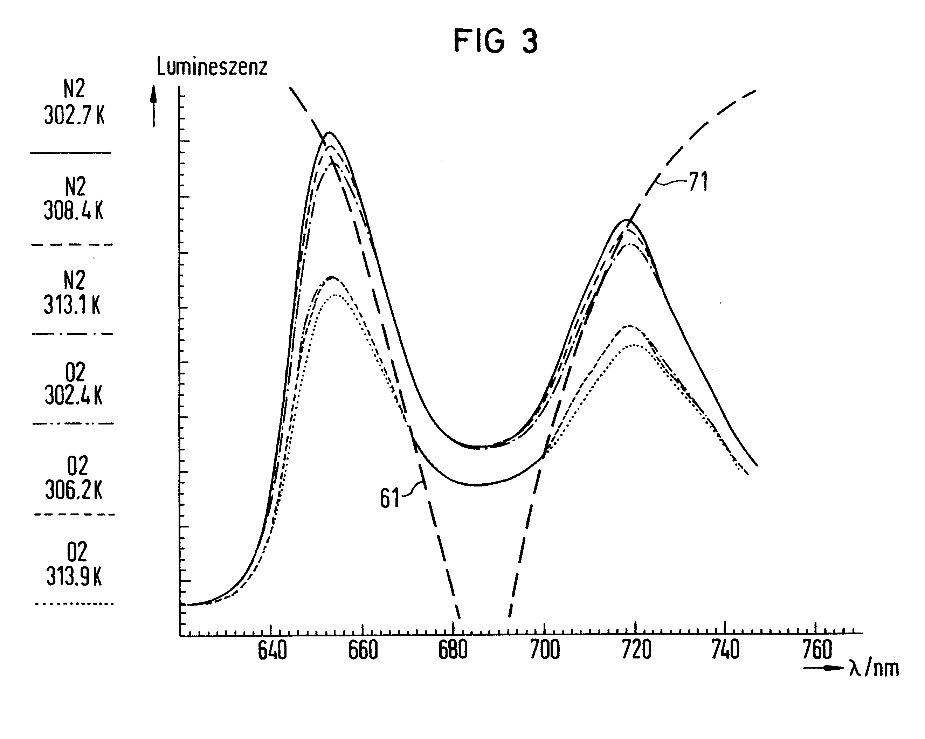

Zum besseren Verständnis ist in der folgenden Fig. 3 das Emissionsspektrum einer lumineszierenden Schicht und dessen Abhängigkeit von den Parametern Sauerstoff und Temperatur dargestellt.Im linken Bereich der Figur sind die Bedingungen für die verschiedenen Kurven angegeben . Das Lumineszenzspektrum weist zwei Emissionsmaxima auf, eins etwa bei 655 und das andere bei 720 nm. Das Anregungslicht

5

hat eine kürzere Wellenlänge. Wie dieser Fig. zu entnehmen ist, tritt durch Sauerstoff eine starke Lumineszenzlöschung ein. Einen gleichgerichteten, jedoch schwächeren Effekt verursacht steigende Temperatur. Darüberhinaus tritt aber mit steigender Temperatur unabhängig von der Sauerstoffkonzentration noch eine Verschiebung der Lumineszenzmaxima zu höheren Wellenlängen hin auf. Da es bei der Respiratorbehandlung von Patienten in der ausgeatmeten Luft zu raschen Temperaturschwankungen über mehrere Grade Celsius kommen kann, war bisher die schnelle Sauerstoffkonzentrationsmessung, beispielsweise in der Exspirationsphase, nicht möglich.

Das erfindungsgemässe Verfahren sieht nun vor, das Lumineszenzlicht in zwei Wellenlängenbereichen zu messen. In Fig.3 sind gestrichelt beispielsweise die obere Bandkante 61 des Filters 6 und die untere Bandkante 71 des Filters 7 angedeutet. Tritt nun z.B. eine Temperaturerhöhung auf, so wandert - anschaulich gesprochen - das erste Lumineszenzmaximum aus dem Transparenzbereich des Filters 6 heraus und das zweite Maximum in den Transparenzbereich des Filters 7 hinein. Wie diese Ueberlegungen zeigen, ergeben sich daher bei einer Temperaturänderung recht unterschiedliche Veränderungen der Signale der Detektoren 10 bzw. 11. Gemäss der Formel für zwei Parameter, in diesem Falle die Sauerstoffkonzentration, die sich bei bekanntem Druck durch den Sauerstoffpartialdruck $p_{O2}$ ausdrücken lässt, und die Temperatur T, ergibt sich folgendes Detektorsignal $S_1$:

$$S_1 = \frac{i_1}{\sqrt{1 + k_1\, p_{O2}}} + b_1 T + a_1$$

Entsprechendes gilt für das Detektorsignal $S_2$. Wenn mit Hilfe verschiedener Kalibrierungen die Koeffizienten ermittelt sind, können aus diesen beiden Gleichungen die Werte für die beiden Parameter T und $p_{O2}$ in der Signalbearbeitungseinrichtung ermittelt werden. Dabei ist zu beachten, dass die Koeffizienten in der Formel für $S_2$ andere Werte annehmen können als in der Formel für $S_1$. Gewisse Koeffizienten müssen dabei durch Kalibrierungen festgelegt werden. Andere können stets feste Werte haben.

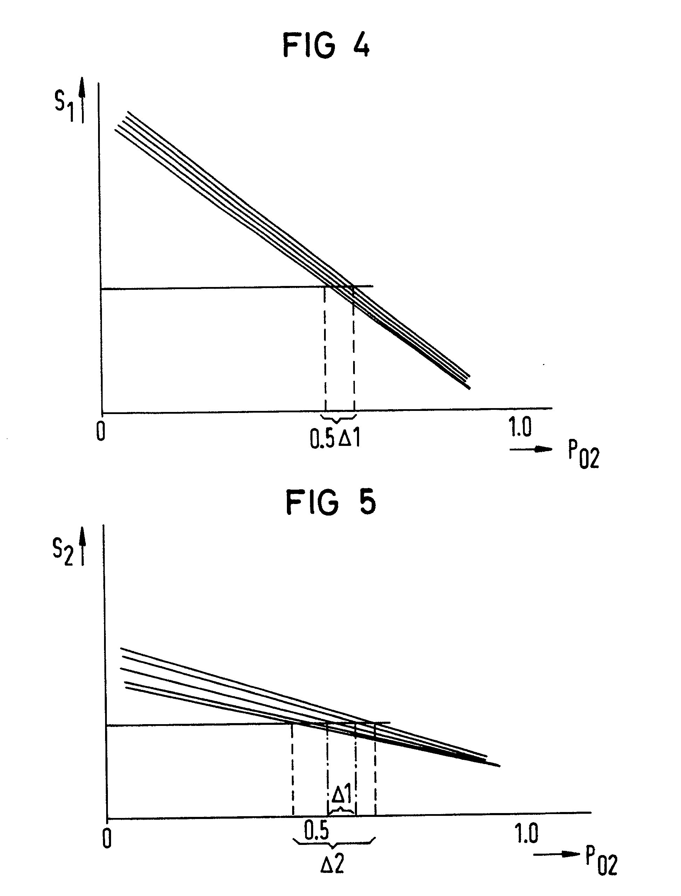

Wie bereits erwähnt, können die beiden Parameter auch iterativ bestimmt werden. Das soll kurz anhand der Figuren 4 und 5 erläutert werden. In beiden Figuren ist der Verlauf der Messignale der Detektoren 10 bzw. 11 in Abhängigkeit vom Sauerstoffpartialdruck aufgetragen und der Einfachheit halber - da es nur um das Prinzip geht - ein linearer Zusammenhang angenommen. Die fünf Geraden in beiden Figuren gelten für fünf verschiedene Temperaturen zwischen 20 und 30 Grad Celsius. Mit steigender Temperatur nehmen die Detektorsignale ab.

Es sei nun angenommen, dass jeder Detektor ein Messignal liefert. Diesen entsprechen, wegen der unbestimmen Temperatur, auf den $P_{O2}$-Achsen ein $\Delta$ 1 bzw $\Delta$ 2 Bereich. Setzt man den kleineren Bereich, im vorliegenden Fall $\Delta$ 1 in das Diagramm gemäss Fig.5 ein, so zeigt sich, dass dann nicht mehr alle Temperaturen zulässig sind. Mit anderen Worten, der mögliche Temperaturbereich wird verkleinert. Dadurch verkleinert sich nun wiederum gemäss Fig.4 der $p_{O2}$ Bereich, mit dem man dann wieder in Fig.5 die Temperatur weiter einschränkt u.s.w.. Durch wiederholtes Anwenden dieses Verfahrens lassen sich also ebenfalls die beiden Parameter mit hinreichender Genauigkeit bestimmen.

Welches Verfahren man jeweils wählen wird, hängt u.a. davon ab, ob sich für bestimmte Parameter, insbesondere wenn es mehr als zwei sind, eine genügend exakte Formel aufstellen lässt und welchen Rechenaufwand man zulassen kann, um noch in wenigen ms die Parameterwerte zu erhalten.

Misst bzw. bestimmt man neben der Sauerstoffkonzentration auch noch die Sauerstoffaufnahme, die $CO_2$-Konzentration, die $CO_2$-Produktion, den Druck, den Gasfluss und den respiratorischen Quotienten, so erhält man ein vollständiges Bild über die bei der Beatmung eines Patienten vorliegenden Verhältnisse.

Das erfindungsgemässe Verfahren und die Anordnung zur Durchführung desselben kann vorteilhaft auch noch für andere Messungen ausgenutzt werden. Ist beispielsweise der prozentuale Sauerstoffgehalt konstant - derartige Verhältnisse liegen in der Erdatmosphäre vor - so variiert die mit der Lumineszenzschicht in Berührung kommende Sauerstoffmenge mit dem Druck. Mit dem Verfahren können in diesem Fall neben der Temperatur der Gasdruck gemessen werden. Die Anordnung stellt daher auch ein ausgezeichnetes Barometer dar.

Wählt man eine Schicht, deren Lumineszenzeigenschaften von Feuchtigkeit abhängen, so kann man in entsprechender Weise ein sehr schnelles und exaktes Hygrometer erhalten.

Durch Wahl der Anregungswellenlänge, der Filtercharakteristika und speziell der Schichteigenschaften lassen sich für alle Messungen gute Bedingungen schaffen.

Mit Hilfe von wasserunempfindlichen Schichten beispielsweise kann mittels eines kleinen Katheters der Blutsauerstoffgehalt schnell bestimmt werden. Integriert in eine Herzschrittmacherelektrode könnte somit ein physiologischer Steuerparameter gemessen werden.

Die Erfindung ist nicht auf die durch die Ausführungsbeispiele angegebenen Verfahren und Anordnungen beschränkt, sondern kann mit Hilfe normalen fachmännischen Könnens in weiten Bereichen variiert werden, ohne den durch die Ansprüche festgelegten Rahmen zu verlassen.So besteht z.B. die Möglichkeit, Lumineszenzschichten zu verwenden, die aus mehreren Kombinationen Farbstoff/Trägermaterial bestehen, wobei jede Kombination auf einen oder einige der interessierenden Parameter reagiert. Die unterschiedlichen Kombinationen können dabei in der Lumineszenzschicht getrennt liegen. Die Anregung kann über eine gemeinsame Lichtquelle oder über veschiedene erfolgen.

## Ansprüche

1. Verfahren zur schnellen Bestimmung mindestens eines Parameters eines Probenmediums, insbesondere eines fliessenden Probenmediums, wobei Licht definierter Wellenlänge, möglicherweise unter Zwischenschalten eines Filters (4), auf mindestens eine lumineszierende Schicht (3) gelenkt wird, die direkt oder indirekt mit dem Probenmedium in Kontakt kommt und deren Lumineszenzeigenschaften von dem Parameter abhängt, und wobei das Lumineszenzlicht über Filter (6,7) auf Detektoren (10,11) gelenkt wird, deren Signale ein Mass für den zu bestimmenden Parameter sind, **dadurch gekennzeichnet,** dass beim Vorliegen weiterer, die Lumineszenzeigenschaften beinflussender Parameter die Intensität für ebensoviele Wellenlängenbereiche bestimmt wird, wie insgesamt Parameter vorhanden sind, wobei die Wellenlängenbereiche so gewählt sind, dass die Parameter die Lumineszenzeigenschaften der Schicht (3) mindestens in einem Wellenlängenbereich unterschiedlich beeinflussen, und dass die so erhaltenen Detektorsignale einer Signalbearbeitungseinrichtung (26) zugeführt werden, die daraus zumindest die Grösse des zu messenden Parameters bestimmt.

2. Verfahren nach Anspruch 1, **dadurch gekennzeichnet,** dass die spektrale Verteilung des auf die lumineszierende Schicht (3) gelenkten Lichtes über einen Regelkreis (12, 15, 16) konstant gehalten wird.

3. Verfahren nach Anspruch 1 oder 2, **dadurch gekennzeichnet,** dass die Intensität des Lichtes konstant gehalten wird.

4. Verfahren nach einem der Anspüche 1 bis 3, **dadurch gekennzeichnet,** dass die Intensität des von der Schicht (3) kommenden Lichtes im Wellenlängenbereich des eingestrahlten Lichtes bestimmt und als Referenzsignal zur Korrektur der Lumineszenzintensitäten verwendet wird.

5. Verfahren nach einem der Ansprüche 1 bis 4, **dadurch gekennzeichnet,** dass das auf die lumineszierende Schicht (3) gelenkte Licht gepulst ist.

6. Verfahren nach einem der Ansprüche 1 bis 5, **dadurch gekennzeichnet,** dass beim Vorliegen zweier die Lumineszenzeigenschaften beeinflussender Parameter $X_1$ und $X_2$ angesetzt wird, dass die Detektorsignale ($S_i$, $i = 1,2$) sich gemäss der Formel

$$ S_i = \frac{i_i}{1 + k_i\, X_1} + b_i\, X_2 + a_i $$

verhalten, wobei $i_i$ die Lumineszenzintensität im Wellenlängenbereich $\lambda_i$ und bei einem bestimmten Wert des Parameter $X_1$, insbesonder $X_i = 0$, $a_i$ eine Untergrundintensität und $k_i$ und $b_i$ Näherungskoeffizienten sind.

7. Verfahren zur Bestimmung der Parameter eines Probenmediums nach einem der Ansprüche 1 bis 6,

7

**dadurch gekennzeichnet,** dass als Parameter $X_1$ der Sauerstoffpartialdruck $P_{02}$ und als $X_2$ die Temperatur T gewählt sind.

8. Anordnung zur Durchführung des Verfahrens nach einem der Ansprüche 1 bis 7, **dadurch gekennzeichnet,** dass als Lichtquelle mindestens eine Leuchtdiode (2) vorgesehen ist, dass ein Diodenparameter bestimmt und zur Regelung der Anssteuerung der Diode(n) (2) derart herangezogen wird, dass die Temperatur des aktiven Diodenbereiches einen definierten Wert bzw. Verlauf erhält, daß das Licht der Diode(n) (2) durch ein Filter (4) auf eine lumineszierende Schicht (3) fällt, dass zwischen der Schicht (3) und weiteren Detektoren (9-11) weitere Filter (5-7) angeordnet sind, dass eine Vorrichtung vorgesehen ist, über die das Probenmedium direkt oder indirekt an die Schicht (3) herangeführt wird und dass die Detektorsignale einer Signalbearbeitungseinrichtung (26) zum Bestimmen der Parameter zugeführt sind.

9. Anordnung nach Anspruch 8, **dadurch gekennzeichnet,** dass eine Anzahl in Reihe geschalteter Leuchtioden (2) vorgesehen sind.

10. Anordnung nach Anspruch 8, **dadurch gekennzeichnet,** dass eine Anzahl parallel geschalteter Leuchtdioden vorgesehen sind.

11. Anordnung nach einem der Ansprüche 8-10, **dadurch gekennzeichnet,** dass die Leuchtdioden gepulst betrieben werden.

12. Anordnung gemäss Anspruch 11, **dadurch gekennzeichnet,** dass der Strom während der Impulse konstant gehalten wird und die Impulsdauer veränderbar ist.

13. Anordnung gemäss Anspruch 11, **dadurch gekennzeichnet,** dass die Stromstärke während der Impulse variierbar ist.

14. Anordnung nach einem der Ansprüche 8-13, **dadurch gekennzeichnet,** dass die Intensität in zwei Wellenlängenbereichen des Diodenspektrums bestimmt und deren Quotient zur Regelung der Ansteuerung der Dioden verwendet wird.

15. Anordnung nach einem der Ansprüche 8-14, **dadurch gekennzeichnet,** dass die Detektoren (9-11) die Intensität während eines Teils der Impulsdauer der Leuchtdioden registrieren.

16. Anordnung nach Anspruch 15, **dadurch gekennzeichnet,** dass die Registrierzeit in Abhängigkeit von einem Referenzsignal geregelt wird.

17. Anordnung nach einem der Ansprüche 8-13,15,16, **dadurch gekennzeichnet,** dass ein Leuchtdiodenparameter wärend des Impulses, insbesondere zu Beginn des Impulses bestimmt und zur Formung desselben herangezogen wird und dass in Abhängigkeit vom Wert des Diodenparameters die zeitliche Lage der Messdauer, in der die Detektoren (9-11) die Lumineszenzintensität messen, geregelt ist.

18. Anordnung nach einem der Ansprüche 8 bis 17, **dadurch gekennzeichnet,** dass die Leuchtdioden (2), Filter (4,5-7), die luminenszierende Schicht (3) und die Detektoren (9-11) in einem lichtdichten Gehäuse (1) angeordnet sind.

## Claims

1. Method for rapidly determining at least one parameter of a sample medium, preferably a flowing sample medium, light of defined wavelength being directed, possibly with the interposition of a filter (4), onto at least one luminescent layer (3), which comes into contact directly or indirectly with the sample medium and whose luminescent properties depend upon the parameter, and the luminescence light being directed via filters (6, 7) onto detectors (10, 11) whose signals are a measure of the parameter to be determined, characterised in that in the presence of further parameters influencing the luminescent properties the intensity is determined for exactly as many wavelength regions as there are parameters present in total, the wavelength regions being selected such that the parameters differently influence the luminescent properties of the layer (3) at least in one wavelength region, and in that the detector

8

signals thus obtained are fed to a signal processing device (26), which determines therefrom at least the magnitude of the parameter to be measured.

2.  Method according to Claim 1, characterised in that the spectral distribution of the light directed onto the luminescent layer (3) is maintained constant via a feedback control loop (12, 15, 16).

3.  Method according to Claim 1 or 2, characterised in that the intensity of the light is maintained constant.

4.  Method according to one of Claims 1 to 3, characterised in that the intensity of the light coming from the layer (3) is determined in the wavelength region of the irradiated light and is used as a reference signal for correcting the luminescence intensities.

5.  Method according to one of Claims 1 to 4, characterised in that the light directed onto the luminescent layer (3) is pulsed.

6.  Method according to one of Claims 1 to 5, characterised in that in the presence of two parameters $X_1$ and $X_2$ influencing the luminescent properties it is set up that the detector signals ($S_i$, $i = 1,2$) behave according to the formula

$$S_i = \frac{i_i}{1 + k_i X_1} + b_i X_2 + a_i$$

where $i_i$ is the luminescence intensity in the wavelength region $\lambda_i$, and at a definite value of the parameter $x_1$, preferably $X_1 = 0$, $a_i$ is a background intensity and $k_i$ and $b_i$ are approximation coefficients.

7.  Method for determining the parameters of a sample medium according to one of Claims 1 to 6, characterised in that the partial oxygen pressure $P_{o2}$ is selected as the parameter $X_1$ and the temperature T is selected as $X_2$.

8.  Arrangement for carrying out the method according to one of Claims 1 to 7, characterised in that at least one light-emitting diode (2) is provided as light source, in that a diode parameter is determined and used to control the driving of the diode(s) (2) in such a way that the temperature of the active diode region is provided with a defined value or characteristic, in that the light of the diode(s) (2) falls through a filter (4) onto a luminescent layer (3), in that further filters (5-7) are arranged between the layer (3) and further detectors (9-11), in that a device is provided via which the sample medium is brought directly or indirectly up to the layer (3), and in that the detector signals are fed to a signal processing device (26) to determine the parameters.

9.  Arrangement according to Claim 8, characterised in that a number of light-emitting diodes (2) are provided connected in series.

10. Arrangement according to Claim 8, characterised in that a number of light-emitting diodes are provided connected in parallel.

11. Arrangement according to one of Claims 8-10, characterised in that the light-emitting diodes are operated in a pulsed fashion.

12. Arrangement according to Claim 11, characterised in that the current is maintained constant during the pulses and the pulse duration is variable.

13. Arrangement according to Claim 11, characterised in that the current strength is variable during the pulses.

14. Arrangement according to one of Claims 8-13, characterised in that the intensity is determined in two wavelength regions of the diode spectrum and its quotient is used to control the driving of the diodes.

15. Arrangement according to one of Claims 8-14, characterised in that the detectors (9-11) record the intensity during a portion of the pulse duration of the light-emitting diodes.

16. Arrangement according to Claim 15, characterised in that the recording time is controlled as a function of a reference signal.

17. Arrangement according to one of Claims 8-13, 15 or 16, characterised in that a light-emitting diode parameter is determined during the pulse, preferably at the beginning of the pulse, and is used to form the latter, and in that the timing of the measurement duration in which the detectors (9-11) measure the luminescence intensity is controlled as a function of the value of the diode parameter.

18. Arrangement according to one of Claims 8 to 17, characterised in that the light-emitting diodes (2), filters (4, 5-7), the luminescent layer (3) and the detectors (9-11) are arranged in a light-tight housing (1).

## Revendications

1. Procédé pour déterminer rapidement au moins un paramètre d'un milieu échantillon, notamment d'un milieu échantillon fluide, selon lequel on dirige une lumière possédant une longueur d'onde définie, éventuellement moyennant l'interposition d'un filtre (4), sur au moins une couche luminescente (3), qui est directement ou indirectement en contact avec le milieu échantillon et dont la caractéristique de luminescence dépend du paramètre, et selon lequel la lumière de luminescence est dirigée, au moyen de filtres (6,7), sur des détecteurs (10,11), dont les signaux représentent une mesure du paramètre devant être déterminé, caractérisé par le fait que, dans le cas de la présence d'autres paramètres influençant les caractéristiques de luminescence, on détermine l'intensité pour un nombre de gammes de longueurs d' onde égal au nombre total de paramètres présents, les gammes de longueurs d'onde étant choisies de manière que les paramètres des caractéristiques de luminescende de la couche (3) aient une influence différente au moins dans une gamme de longueurs d'onde, et que les signaux ainsi obtenus des détecteurs sont envoyés à un dispositif (26) de traitement des signaux, qui détermine, à partir de ces signaux, au moins la grandeur des paramètres à mesurer.

2. Procédé suivant la revendication 1, caractérisé par le fait que la distribution spectrale de la lumière dirigée sur la couche luminescente (3) est maintenue constante par l'intermédiaire d'un circuit de régulation (12,15,16).

3. Procédé suivant la revendication 1 ou 2, caractérisé par le fait que l'intensité de la lumière est maintenue constante.

4. Procédé suivant l'une des revendications 1 à 3, caractérisé par le fait qu'on détermine l'intensité de la lumière, qui arrive de la couche (3), dans la gamme des longueurs d'onde de la lumière incidente et qu'on l'utilise comme signal de référence pour corriger les intensités de la luminescence.

5. Procédé suivant l'une des revendications 1 à 4, caractérisé par le fait que la lumière envoyée sur la couche luminescente (3) est pulsée.

6. Procédé suivant l'une des revendications 1 à 5, caractérisé par le fait que, dans le cas de la présence de deux paramètres $x_1$ et $x_2$ influençant les caractéristiques de luminescence, on suppose que les signaux ($S_i$, $i = 1, 2$) des détecteurs se comportent conformément à la formule

$$S_i = \frac{i_i}{1 + k_i x_1} + b_i x_2 + a_i$$

$i_i$ représentant l'intensité de la luminescence dans la gamme des longueurs d'ondes $\lambda_i$ et pour une

valeur déterminée des paramètres $X_1$, notamment $X_i = 0$, $a_i$ désignant une intensité du rayonnement ambiant et $k_i$ et $b_i$ des coefficients d'approximation.

7. Procédé pour déterminer les paramètres d'un milieu échantillon suivant l'une des revendications 1 à 6, caractérisé par le fait qu'on choisit, comme paramètre $X_1$, la pression partielle d'oxygène $P_{02}$ et, comme paramétre $X_2$, la température T.

8. Dispositif pour la mise en oeuvre du procédé suivant l'une des revendications 1 à 7, caractérisé par le fait qu'on prévoit, comme source de lumière, au moins une diode à luminescence (2), qu'on détermine un paramètre de la diode et qu'on l'utilise pour la régulation de la commande de la ou des diodes (2) de manière que la température de la zone active de la diode prenne une valeur ou une allure définie, que la lumière de la ou des diodes (2) rencontre la couche luminescente (3) après avoir traversé un filtre (4), que d'autres filtres (5-7) sont disposés entre la couche (3) et d'autres détecteurs (9-11), qu'il est prévu un dispositif permettant d'approcher directement ou indirectement le milieu échantillon de la couche (3) et que les signaux des détecteurs sont envoyés à un dispositif (26) de traitement des signaux pour la détermination des paramètres.

9. Dispositif suivant la revendication 8, caractérisé par le fait qu'il est prévu un certain nombre de diodes à luminescence (2) branchées en série.

10. Dispositif suivant la revendication 8, caractérisé en ce qu'il est prévu un certain nombre de diodes à luminescence branchées en parallèle.

11. Dispositif suivant l'une des revendications 8-10, caractérisé par le fait que les diodes à luminescence fonctionnent d'une manière pulsée.

12. Dispositif selon la revendication 11, caractérisé en ce que le courant est maintenu constant pendant les impulsions et que la durée des impulsions peut être modifiée.

13. Dispositif selon la revendication 11, caractérisé en ce que l'intensité du courant peut être modifiée pendant les impulsions.

14. Dispositif selon l'une des revendications 8-13, caractérisé par le fait qu'on détermine l'intensité dans deux gammes de longueurs d'onde du spectre des diodes et qu'on utilise le quotient de ces intensités pour régler la commande des diodes.

15. Dispositif suivant l'une des revendications 8-14, caractérisé par le fait que les détecteurs (9-11) enregistrent l'intensité pendant une partie de la durée des impulsions des diodes à luminescence.

16. Dispositif selon la revendication 15, caractérisé en ce que la durée d'enregistrement est réglée en fonction d'un signal de référence.

17. Dispositif suivant l'une des revendications 8-13, 15, 16, caractérisé par le fait qu'un paramètre des diodes à luminescence est déterminé pendant l'impulsion, notamment au début de l'impulsion, et est utilisé pour la formation de ces impulsions et que la position temporelle de la durée de mesure, pendant laquelle les détecteurs (9-11) mesurent l'intensité de la luminescence, est réglée en fonction de la valeur du paramètre des diodes.

18. Dispositif selon l'une des revendications 8 à 17, caractérisé par le fait que les diodes à luminescence (2), les filtres (4,5-7), la couche luminescente (3) et les détecteurs (9-11) sont disposés dans un boîtier (1) étanche à la lumière.

FIG 1

FIG 2

FIG 3

## FIG 4

## FIG 5